Europäisches Patentamt

European Patent Office

Office européen des brevets

(11) Publication number: **0 386 236 A1**

## EUROPEAN PATENT APPLICATION
### published in accordance with Art. 158(3) EPC

(21) Application number: 88907384.7

(51) Int. Cl.5: **A61B 1/00**

(22) Date of filing: 30.08.88

(86) International application number:
**PCT/JP88/00860**

(87) International publication number:
**WO 89/01755 (09.03.89 89/06)**

(30) Priority: 01.09.87 JP 218855/87
25.05.88 JP 127351/88

(43) Date of publication of application:
**12.09.90 Bulletin 90/37**

(84) Designated Contracting States:
**BE DE FR**

(71) Applicant: **Terumo Kabushiki Kaisha**
**No. 44-1, Hatagaya 2-chome Shibuya-ku**
**Tokyo 151(JP)**

(72) Inventor: **HONDA, Hiroaki**
**1-31, Sobe 1-chome**
**Naha-shi Okinawa 900(JP)**

(74) Representative: **Casalonga, Axel et al**
**BUREAU D.A. CASALONGA - JOSSE**
**Morassistrasse 8**
**D-8000 München 5(DE)**

(54) CATHETER TUBE.

(57) This invention relates to a catheter tube to be inserted into and allowed to stay in a body cavity having a relatively small diameter as typified by the blood vessel and used for observation or medical treatment of the interior of the body cavity. The catheter tube of the present invention has a structure wherein a linear inspection or medical instrument in the form of an optical fiber bundle is stored in a lumen formed in a tube body and is characterized in that the tip of the inspection or medical instrument is bent by moving the instrument in the longitudinal direction of the tube without bending substantially the tip of the catheter tube. Accordingly, the inspection and medical treatment of the inner wall of the body cavity can be conducted with a simple structure and the diameter of the catheter tube can be reduced.

FIG. 1

-1-

## DESCRIPTION

### TITLE OF THE INVENTION
Catheter Tube

### FIELD OF THE INVENTION

This invention relates to a catheter for use by inserting into a body cavity for visualizing the interior of the body cavity and/or medically treating the inner wall of the body cavity from without the body, and more particularly, to such a catheter tube constructing an endoscope or fiberscope.

### BACKGROUND OF THE INVENTION

Endoscopes are inserted into a body cavity from outside the body to visualize the interior of the body cavity while allowing for medical treatments including injection of medicament fluid to the cavity wall and exposure to laser beams. They recently attract greater interest encouraging their development efforts.

Endoscopes are of a configuration wherein a bundle of optical fibers for transmitting and receiving light is accommodated in a flexible catheter tube. The catheter tube is inserted into a blood vessel until it reaches a destined site where the light-transmitting fiber (or light guide) projects light from its distal end to an area under observation and the light-receiving fiber (or image fiber) receives the reflected light at the distal end and conducts it to an imaging section where a visual image is provided for observation.

When the endoscope is used to visualize the interior of the body cavity, the catheter tube and the optical fiber bundle extend parallel to the body cavity. If it is desired to visualize a direction other than forward of the distal end of the tube in the cavity (for example, the inner wall of the body cavity), a distal portion of the tube must be bent or curved.

Methods for curving the tube distal portion from without the body included (1) use of a guide wire separate from the catheter tube and (2) use of a plurality of operating wires accommodated in the catheter tube.

However, method (1) has several drawbacks that since the guide wire is essentially intended for guiding the catheter tube to the destined site, it cannot be readily manipulated so as to curve the tube distal portion, that it is difficult to maintain the curve unchanged during the visualizing time, and that a distal portion of the guide wire is soft or limp for its purpose so that it is less effective in turning the catheter tube.

The catheter tube (flexible tube for an endoscope) used in method (2) includes a bendable portion having a plurality of nodal rings serially connected for turning motion and a plurality of operating wires having one end connected to the bendable portion whereby the bendable portion is bent by pulling any one of the operating wires toward the proximal end by means of a wire manipulator including a pulley and an angle dial at the tube proximal end. (See Japanese Patent Publication No. 21734/1985, and Japanese Utility Model Publication Nos. 23442/1987 and 23447/1987.)

The distal portion of the catheter tube is bent not for the purpose of visualizing the inner wall of the body cavity into which the tube is inserted, but for the purpose of guiding the distal portion of the catheter tube until it reaches the destination. Therefore, the tube distal portion is bent at a relatively large angle. It is also necessary to provide the nodal ring assembly at the tube distal portion as well as operating wires and a device for pulling the wires, resulting in a complex structure and a large tube diameter. Although the bendable portion of the tube has to be soft, the portion of the tube not to be bent (on a side proximal of the bendable portion) should be formed of a tube-forming material having hardness (for example, polyvinyl chloride, polyamide, polyurethane, polytetrafluoroethylene, tetrafluoroethylene-hexafluoropropylene polymer and the like) to insure stiffness so that the softness of the overall tube is limited. Therefore, this tube can be used only in a body cavity having a relatively large inner diameter such as digestive and bronchial systems. For the endoscope which is used by inserting into a body cavity having a relatively small inner diameter such as blood vessels and ureters, there is currently available no appropriate method for bending a tube distal portion of the endoscope from without the body.

An approach which does not require to bend the distal end of a catheter tube is to visualize the wall of a body cavity by mounting a small prism at the distal end of an optical fiber bundle in the catheter tube so that the visual angle is variable. This approach uses a complex optical system which is not easy to manufacture and requires a greater expense. The catheter tube of this construction is difficult to reduce in diameter and thus least applicable to the endoscope for visualizing a body cavity having a small diameter such as blood vessels and ureters.

## DISCLOSURE OF THE INVENTION

An object of the present invention is to eliminate the above-mentioned drawbacks of the prior art and to provide a catheter tube of a simple structure capable of visualizing or medically treating the inner wall of a body cavity having a small diameter such as blood vessels.

To attain the above and other objects, the inventors have found through extensive investigations that a catheter tube can be simplified in structure and reduced in diameter by bending a distal portion of an optical fiber bundle (or visualizing or therapeutic instrument) itself rather than bending a distal portion of the catheter tube. The present invention is predicated on this finding.

As a result of further investigations, the inventors have discovered the following first, second and third forms of catheter tube.

The first form of the invention is a catheter tube in which a bundle of optical fibers is accommodated in a lumen in a tube body for motion in an axial direction of the tube body, and movement of the optical fiber bundle toward the distal end of the tube causes a distal portion of the optical fiber bundle to slide along the ramp surface of a guide member, thereby causing the distal end of the optical fiber bundle to protrude from a side port in the outer peripheral wall of the tube body so as to point to the inner wall of the body cavity.

The second form of the invention is a catheter tube in which an optical fiber bundle is accommodated in a lumen in a tube body for motion in an axial direction of the tube body, and movement of the optical fiber bundle toward the distal end of the tube causes a distal portion of the optical fiber bundle to slide along a bending section in a distal portion of the lumen, thereby causing the distal end of the optical fiber bundle to turn a predetermined angle so as to point to the inner wall of the body cavity.

The third form of the invention is a catheter tube in which an optical fiber bundle having a previously bent distal portion is accommodated in a lumen in a tube body for motion in an axial direction of the tube body such that bending of the optical fiber bundle distal portion is restrained by the lumen inner wall when the optical fiber bundle distal portion is within the lumen. When the optical fiber bundle is moved toward the tube distal end until the optical fiber bundle distal end is protruded out of the lumen, such restraint is released to allow the optical fiber bundle distal portion to resume the bend so that the optical

-5-

fiber bundle distal end points to the inner wall of the body cavity.

The present invention does visualize or medically treat the inner wall of the body cavity without bending the distal portion of the catheter tube. When it is applied to a blood vessel endoscope in the form of a balloon catheter, inflation of the balloon ensures anchoring of the catheter tube to the body cavity (or blood vessel) and blocking of a blood flow. Further, since it is not necessary to provide the tube body with a bent portion distal of the balloon, the balloon can be located at a more distal side of the tube body so that blood can be more effectively removed by flushing a smaller amount of clear liquid.

As understood from the foregoing description, the first form of the present invention provides a catheter tube capable of accommodating therein a wire-like visualizing or therapeutic instrument, the catheter tube being provided with a bending mechanism for bending a distal portion of the visualizing or therapeutic instrument protruding from a distal portion of the catheter tube, without substantially bending the distal portion of the catheter tube.

The second form of the present invention provides a catheter tube for use by inserting into a body cavity, comprising

a tube body,

a side port defined in the outer peripheral wall of the tube body adjacent a distal end thereof,

a first lumen in flow communication with the side port,

a visualizing or therapeutic instrument accommodated for movement in the first lumen,

a guide member mounted in the first lumen of the tube body adjacent its distal end, the guide member having a ramp surface for guiding a distal portion of the visualizing or therapeutic instrument from within the first lumen to the side port when the visualizing or therapeutic instrument is moved toward the distal end of the tube body,

a second lumen open at the distal end of the tube body, and

a moving mechanism attached to a proximal portion of the tube body for axially moving the visualizing or therapeutic instrument through the tube body.

One preferred embodiment of the second form is
(i)  the catheter tube wherein the ramp surface has an average inclination angle of 5 to 80° relative to the axial direction of the tube body.

The third form of the present invention provides a catheter tube for use by inserting into a body cavity, comprising

a tube body,

a first lumen open at the distal end of the tube body, the first lumen at a distal portion thereof having a bending section which is inclined relative to the axial direction of the tube body,

a visualizing or therapeutic instrument accommodated for movement in the first lumen,

a second lumen open at the distal end of the tube body, and

a moving mechanism attached to a proximal portion of the tube body for axially moving the visualizing or therapeutic instrument through the tube body.

Preferred embodiments of the third form include
(ii)  the catheter tube wherein the bending section of the first lumen has a length of 1 to 20 mm from the distal end of the tube body; and
(iii)  the catheter tube wherein the bending section of the first lumen has an average inclination angle of 5 to 80° relative to the axial direction of the tube body.

The fourth form of the present invention provides a catheter tube for use by inserting into a body cavity, comprising

a tube body,

a first lumen open at the distal end of the tube body,

a visualizing or therapeutic instrument accommodated for movement in the first lumen, a distal portion of the visualizing or therapeutic instrument being bendable when the distal portion of the visualizing or therapeutic instrument is protruded from the open end of the first lumen,

a second lumen open at the distal end of the tube body, and

a moving mechanism attached to a proximal portion of the tube body for axially moving the visualizing or therapeutic instrument through the tube body.

Preferred embodiments of the fourth form include

(iv) the catheter tube wherein a core member having a previously bent distal portion is embedded in the visualizing or therapeutic instrument so that the distal portion of the visualizing or therapeutic instrument is bent.

(v) the catheter tube wherein the visualizing or therapeutic instrument is bendable over a length of 1 to 20 mm from its distal end.

Preferred embodiments of the first, second, third and forth forms include

(vi) the catheter tube wherein the inner surface of the first lumen has been subjected to lubricating treatment;

(vii) the catheter tube wherein the visualizing or therapeutic instrument is a bundle of optical fibers;

(viii) the catheter tube which further comprises at least one inflatable/contractible balloon attached around the outer peripheral wall of the tube body near its distal end, and a third lumen defined in the tube body in flow communication with the balloon; and

(ix) the catheter tube wherein the balloon, when inflated, has a minimum diameter at least substantially equal to the maximum inner diameter of the body cavity into which the catheter tube is inserted.

-8-

## BRIEF DESCRIPTION OF THE DRAWINGS

FIG. 1 is a fragmental axial cross section of a catheter tube according to one embodiment of the invention.

FIG. 2 is a cross section taken along lines II-II in FIG. 1.

FIG. 3 is a fragmental axial cross section of a proximal portion of the catheter tube of the invention.

FIGS. 4 and 5 are fragmental cross sections showing the catheter tube of FIG. 1 during operation.

FIG. 6 is a fragmental axial cross section of a catheter tube according to another embodiment of the invention.

FIG. 7 is a cross section taken along lines VII-VII in FIG. 6.

FIGS. 8 and 9 are fragmental cross sections showing the catheter tube of FIG. 6 during operation.

FIG. 10 is a fragmental axial cross section of a catheter tube according to a further embodiment of the invention.

FIG. 11 is a cross section taken along lines XI-XI in FIG. 10.

FIGS. 12 and 13 are fragmental cross sections showing the catheter tube of FIG. 10 during operation.

## BEST MODE FOR CARRYING OUT THE INVENTION

The catheter tube of the present invention will be better understood from the following detailed description of its preferred embodiments taken in conjunction with the accompanying drawings. It is to be noted that although the following description refers to the following first, second and third embodiments of the invention as its preferred embodiments and to the typical application of the catheter tube as an endoscope, the invention is not limited to these embodiments and application.

[First Embodiment]

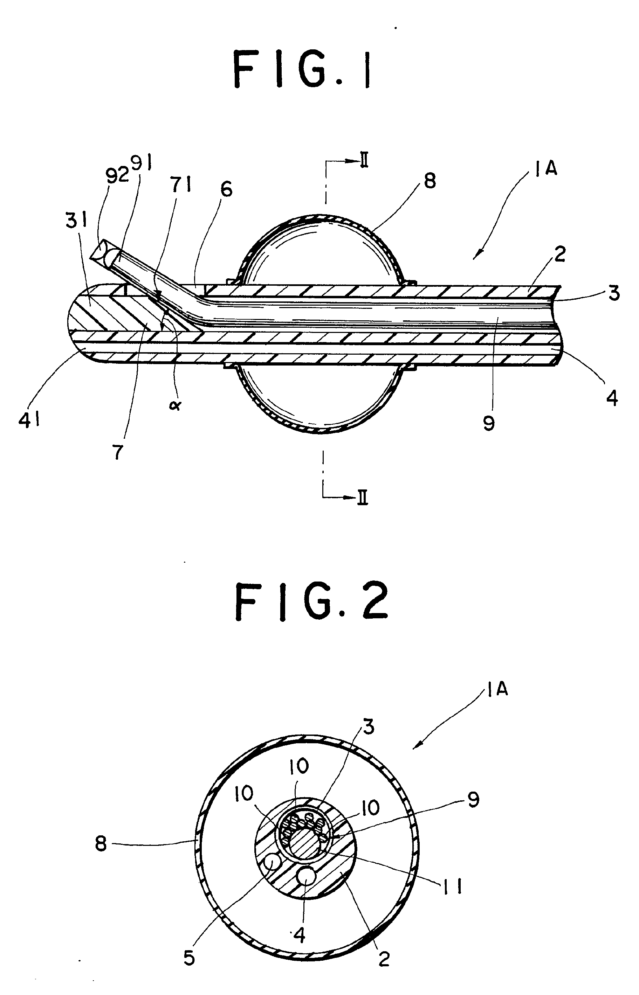

FIG. 1 is a fragmental axial cross-sectional view illustrating the construction of a catheter tube according to the present invention, and FIG. 2 is a cross-section taken along lines II-II in FIG. 1.

As shown in the figures, the catheter tube 1A includes a tube body 2 and a balloon 8 attached around the outer peripheral wall of the tube body 2 near its distal end (on the left side in FIG. 1). The balloon 8 is formed of a rubbery material such as silicone rubber and latex rubber, urethane, polyvinyl chloride, ethylene-vinyl acetate copolymer or the like so that it is inflatable and contractible.

The tube body 2 is formed of a flexible material, for example, polyvinyl chloride, polyurethane, silicone rubber, polyethylene, nylon, ethylene-vinyl acetate copolymer or the like.

Since the catheter tube 1A of the invention is generally used by inserting and leaving it in a body cavity, it is often necessary to carry out visualization or medical treatment while identifying the location of the catheter tube by X-ray radiation. It is thus preferred to impart radiopaqueness to the catheter tube 1A. More particularly, a radiopaque agent is preferably contained in the material of which the tube body 2 and/or the balloon 8 is made. The radiopaque agents include barium sulfate, bismuth oxide, and tungsten, for example.

The tube body 2 is formed with various lumens intended for different purposes and functions as will be described below.

A first lumen 3 has a bundle of optical fibers 9 accommodated therein for axial movement through the tube body. The optical fiber bundle 9 serves as an instrument for visualizing the inner wall of a body cavity when the catheter tube 1A is used as an endoscope. The optical fiber bundle 9 may also be used for a medical treatment such as irradiation of a laser beam to the body cavity inner wall.

The optical fiber bundle 9 includes a light-transmitting fiber (or light guide) 10 and a light-receiving fiber (or image fiber) 11, which are bonded with a binder resin such as acrylic resin, epoxy resin, and silicone into a bundle as shown in FIG. 2. A lens 92 is mounted to the distal end of the optical fiber bundle 9, and the distal portion of the optical fiber bundle 9 is located near a ramp surface 71 of a guide member 7 to be described later.

Light generated by a light source (not shown) at a proximal side (on the right side in FIG. 1) of the catheter tube is conducted through the light-transmitting fiber 10 and projected from the distal end thereof toward a site to be visualized, and the reflected light is taken into the light-receiving fiber 11 at its distal end whereby the image is transmitted through the fiber 11 to an imaging section (not shown) at the proximal side of the catheter tube.

These light-transmitting and receiving fibers are optical fibers formed of quartz, plastics, multi-component glass or the like.

For smooth movement of the optical fiber bundle 9 through the first lumen 3, the inner surface of the first lumen 3 is preferably subjected to a lubricating treatment as by silicone coating, Teflon coating and hydrophilic resin coating.

As shown in FIG. 1, the guide member 7 having the ramp surface 71 is mounted in a distal portion 31 of the first lumen 3 to block the first lumen distal portion 31. The first lumen distal portion 31 need not be necessarily blocked with the guide member 7, but it may permit passage of fluid therethrough.

At a position facing the ramp surface 71 of the guide member 7 (above the ramp surface 71 in FIG. 1), the outer peripheral wall of the tube body is formed with a side port 6 for providing flow communication between the first lumen 3 and the exterior.

When the optical fiber bundle 9 is moved toward the distal end of the tube body by a moving manipulator 12 to be described later, the distal end 91 of the optical fiber bundle 9 abuts against the ramp surface 71 of the guide member 7, is bent thereby, then slides up along the ramp surface 71, is guided to the side port 6, moves past the side port 6, and eventually protrudes out of the tube body 2. The term "bending" is used herein to encompass the concepts that the optical fiber bundle or similar member is bent at one point or at plural points in a broken line form and that it is continuously curved in a curvilinear form.

Since the visible angle of the optical fiber bundle is substantially determined by the average inclination angle $\alpha$ of the ramp surface 71 of the guide member 7 (with respect to the axial direction of the tube body), the average inclination angle $\alpha$ of the ramp surface 71 is preferably in the range of 5 to 80°. The reason is that an angle $\alpha$ of less than 5 degrees would cause the side port 6 to have an increased open area and sometimes fails to fully visualize the inner wall of the blood vessel whereas an angle $\alpha$ of more than 80 degrees would cause the optical fiber bundle to be bent at such a large angle that the optical fiber can be broken.

The ramp surface 71 of the guide member 7 preferably has a length of about 3 to 5 mm and an average inclination angle $\alpha$ of about 10 to 45° particularly when the catheter tube of the invention is applied as an endoscope for visualizing or medically treating a slender body cavity such as a blood vessel and ureter.

The ramp surface 71 is not limited to a planar surface as shown in FIG. 1, and it may be a cylindrical or curved surface.

The side port 6 may be of a sufficient size to permit passage of the distal end 91 of the optical fiber bundle while its shape and other factors are not particularly limited.

The guide member 7 is preferably a separate member made of a resin such as polyvinyl chloride, polyurethane, and ethylene-vinyl acetate copolymer, which is mounted to the distal portion 31 of the first lumen 3. The guide member 7, however, may be either concurrently formed with the tube body 2 by integral molding or subjected to secondary forming (heat fusion).

In order that the distal end 91 of the optical fiber bundle may smoothly slide thereon, the ramp surface 71 may be surface finished or treated (as by silicone coating and hydrophilic resin coating).

A second lumen 4 is open at the distal end of the tube body 2 and serves to admit a fluid from an opening 41 into the body cavity or suck in a fluid from the body cavity. More particularly, the lumen 4 is used to administer a liquid medicament into the body cavity where the catheter tube 1A is inserted and left. When the interior of a blood vessel is visualized by an endoscope, the lumen 4 is used as a flush channel for injecting a clear liquid (e.g., physiological saline and glucose liquid) to displace the visibility impeding blood.

A third lumen 5 is in flow communication with the interior of the balloon 8 as shown in FIG. 2 and serves to feed a fluid into the balloon 8 for inflation or discharge the fluid from the balloon 8 for contraction. The balloon inflating fluids include $CO_2$ gas, liquids such as physiological saline, and liquid radiopaque agents.

The balloon 8 attached to the tube body 2 proximal of the side port 6 is inflated so as to make close contact with the inner wall of the body cavity, thereby playing the role of securing the catheter tube 1A relative to the body cavity and the other role of blocking a further flow of blood beyond the balloon 8 (toward the distal end of the tube) when the visibility impeding blood is purged or displaced by a clear liquid.

The balloon 8 is preferably designed so as to expand radially from the center of the tube body 2 when inflated. The transverse cross section of balloon 8 may have a circular, elliptical or similar shape, but preferably a shape conforming to the transverse cross section of the body cavity where the catheter tube is inserted and indwelled because more tight contact with the body cavity is available. To ensure tight fit of the balloon 8 within the body cavity where the catheter tube is inserted and indwelled, the balloon 8 is preferably dimensioned so as to meet the relationship: $Dmin \geq dmax$ wherein $Dmin$ is the minimum diameter, that is, the diameter of a portion of the balloon which is minimum in a radial direction of the tube when the balloon 8 is inflated and $dmax$ is the maximum diameter, that is, the diameter of a portion of the inner wall of the body cavity which is maximum (when the body cavity is contracted).

The tube body 2 may be provided with a plurality of longitudinally spaced such balloons. The balloon 8 should be attached to the tube body 2 in a gas or liquid tight manner and the possible attachment methods involve adhesive bonding of a separate member (an annular or bag-shaped rubber member or the like), tying with thread, integral molding or two-color molding together with the tube, and any other methods capable of gas or liquid tight attachment of the balloon.

It is also contemplated in the present invention to form one or more lumens in addition to the foregoing first, second and third lumens. Such an additional lumen or lumens are used for various purposes of, for example, inflating an additional balloon if any, injecting a liquid other than the clear liquid, and sucking in blood.

It is also possible to provide a branch lumen diverted from the first lumen 3. In this case, a continuous flow of a small volume of physiological saline from the branch lumen to the first lumen can prevent back entry of blood from the side port 6 at the distal end.

It is possible to omit any one of the first, second and third lumens and to utilize one of the remaining lumens for multiple purposes. For example, when the second lumen 4 is omitted, the first lumen 3 can serve for the double purposes of accommodating the fiber bundle and injecting the clear liquid.

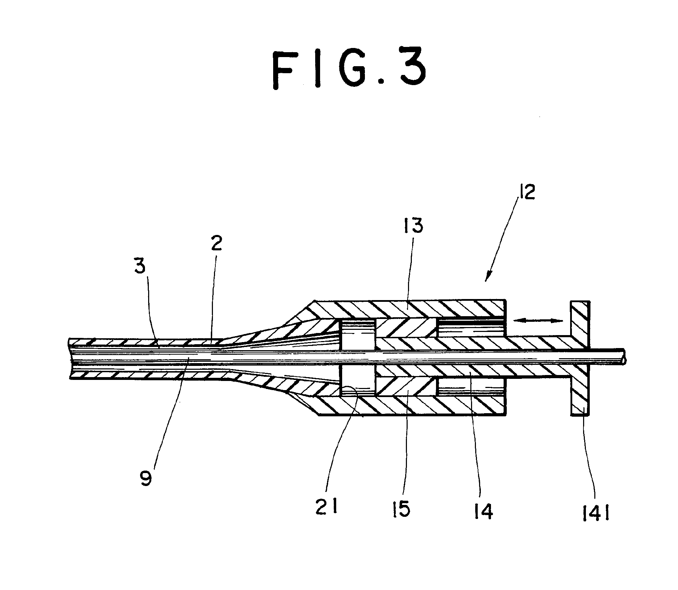

The catheter tube 1A of the invention at a proximal portion is provided with a moving manipulator 12 for moving the optical fiber bundle 9 through the first lumen 3 in an axial direction of the tube body as shown in FIG. 3.

The moving manipulator 12 is a cylindrical connector including a female connector segment 13 having one end connected to the proximal end of the tube body 2 and a piston-shaped male connector segment 14 inserted in the bore of the female connector segment 13 through a guide 15 in the form of a rubber ring. The optical fiber bundle 9 is secured to the male connector segment 14. When the male connector segment 14 is moved axially (in the direction of an arrow in FIG. 3), the optical fiber bundle 9 is accordingly moved through the first lumen 3 in the axial direction of the tube body.

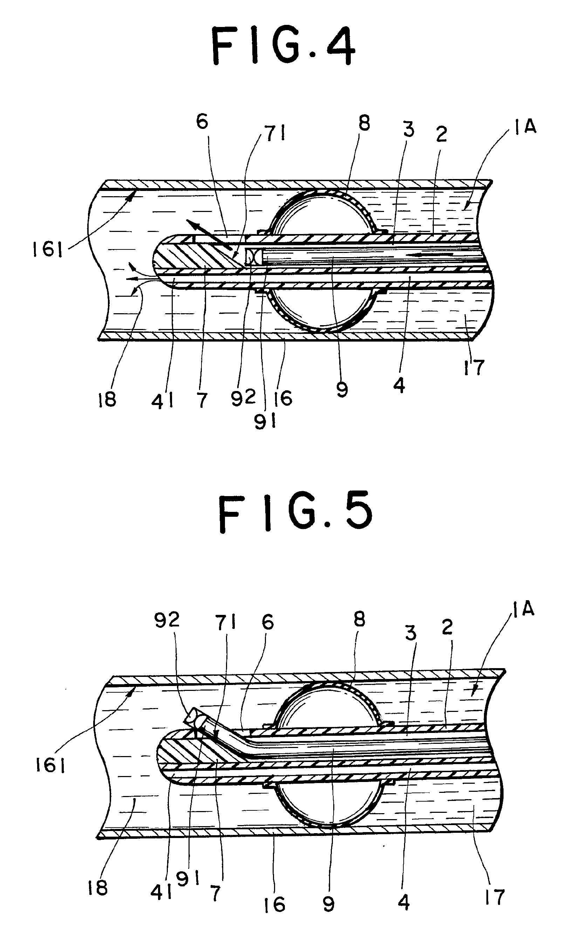

The distance of travel is determined to such a range that the distal end 91 of the optical fiber bundle is moved from the position where it is retracted within the first lumen 3 (the state shown in FIG. 4) to the position where the distal end 91 is protruded a predetermined length from the side port 6 (the state shown in FIG. 5) or the position where the distal end 91 is kept substantially flush with the side port 6.

If the distal end 91 of the optical fiber bundle 9 protrudes too much from the side port 6, it can contact and damage the body cavity. For this reason, the securing location of the optical fiber bundle is adjusted such that the distal end 91 of the optical fiber bundle protrudes a predetermined (or safe) distance from the side port 6 when the guide 15 comes in abutment with the proximal end face 21 of the tube body 2. Alternatively, the male connector segment 14 may be provided with a scale for indicating the distance of projection of the optical fiber bundle from the side port 6.

To limit the stroke of the optical fiber bundle 9, the female connector segment 13 on the inside may be provided with a stop (not shown).

Next, the operation of the catheter tube 1A is described.

FIGS. 4 and 5 are fragmental cross-sectional views showing the catheter tube 1A of the invention during its use.

As shown in FIG. 4, the catheter tube 1A functioning as an endoscope is inserted into a body cavity, that is, a blood vessel 16. A fluid is introduced into the balloon 8 through the third lumen 5 to inflate the balloon 8, which comes in close contact with the inner wall 161 of the blood vessel 16, thereby retaining the catheter tube 1A relative to the blood vessel 16 and blocking further flow of blood 17 through the blood vessel 16.

Further, a clear liquid 18 such as physiological saline is introduced into the second lumen 4 from the proximal side and injected from the distal opening 41, thereby purging or displacing the blood which has been present forward of the balloon 8 (on the distal side of the tube) and filling the volume with the clear liquid 18 instead.

Insofar as the minimum diameter of the balloon 8 when inflated is at least substantially equal to the maximum inner diameter of the blood vessel 16, the balloon 8 makes sufficiently tight contact with the inner wall 161 of the blood vessel 16 to prevent the blood 17 rearward of the balloon 8 (on the proximal side of the tube) from flowing forward of the balloon 8, that is, to a site under observation, ensuring a clear image for observation.

Next, by grasping an operating flange 141 of the moving manipulator 12 as shown in FIG. 3 and manipulating the male connector segment 14, the optical fiber bundle 9 is moved through the first lumen 3 toward the distal end of the catheter tube 1A (in the direction of an arrow in FIG. 4). As the optical fiber bundle 9 is moved forward, the distal end 91 of the optical fiber bundle 9 abuts against the ramp surface 71 of the guide member 7, is bent thereby, slides up along the ramp surface 71, is guided toward the side port 6, moves past the side port 6, and protrudes out of the tube body 2 as shown in FIG. 5.

At this point, the distal end 91 of the optical fiber bundle 9 points to the inner wall 161 of the blood vessel 16 so that the blood vessel inner wall 161 can be visualized through the light-transmitting and receiving fibers 10 and 11.

The blood vessel inner wall 161 can be visualized over its entire circumference by rotating the catheter tube 1A itself within the blood vessel 16.

When the distal end portion 91 of the optical fiber bundle 9 is bent, the distal portion of the tube body 2 can also be somewhat bent due to the reaction force of the bundle, but to a negligibly small extent, giving no adverse influence.

The feature of the present invention is that the distal portion of the optical fiber bundle can be bent without positively bending the distal portion of the catheter tube, but it is not precluded that the distal portion of the catheter tube is somewhat bent as a result of bending of the distal portion of the optical fiber bundle.

-17-

[Second Embodiment]

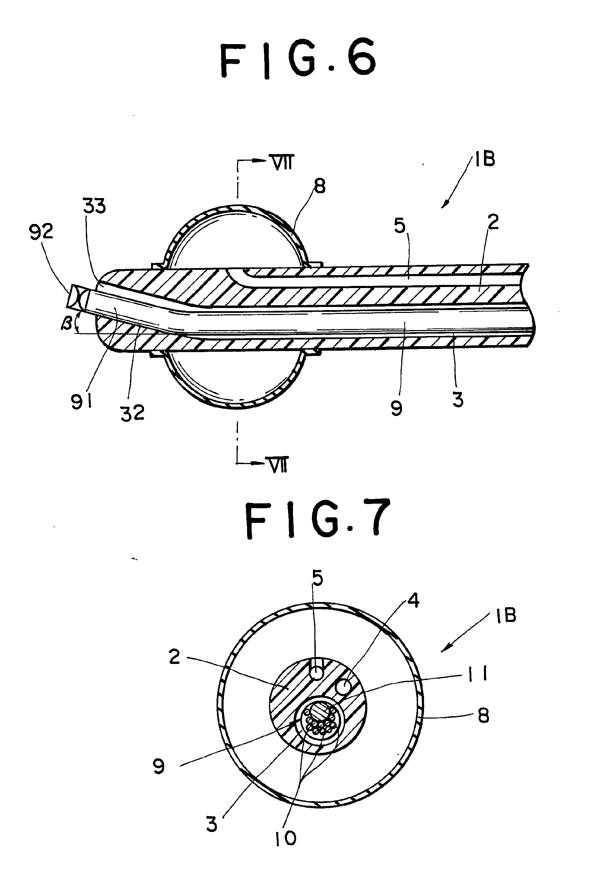

FIG. 6 is a fragmental axial cross-sectional view of a catheter tube according to another embodiment of the present invention, and FIG. 7 is a cross-sectional view taken along lines VII-VII in FIG. 6.

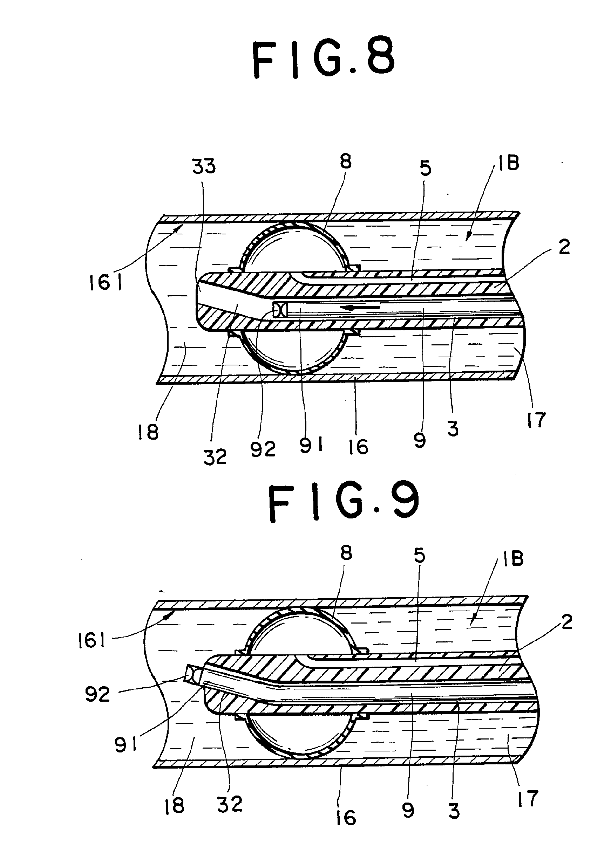

FIGS. 8 and 9 are fragmental cross-sectional views showing the catheter tube 1B of FIG. 6 during its use.

In the description of the construction and operation of the catheter tube 1B shown in these figures, only the difference from the catheter tube 1A of the first embodiment is described because the remaining elements are the same.

The tube body 2 has the first lumen 3 defined therein. In a distal portion of the first lumen 3 (on the left side in FIG. 6) is formed a bending section 32 which is inclined at a predetermined angle with respect to an axial direction of the tube body 2.

When the optical fiber bundle 9 is moved from the position where the distal end 91 of the optical fiber bundle 9 is retracted proximal of the bending section 32 of the first lumen 3 as shown in FIG. 8, toward the distal end of the tube body as shown by an arrow by means of a moving manipulator 12 located at a proximal side of the tube body, the distal end 91 of the optical fiber bundle 9 abuts against the bending section 32, bends thereby, slides up along the bending section 32, and reaches the distal opening 33 of the first lumen 3 or protrudes a predetermined distal out of the distal opening 33 as shown in FIGS. 6 and 9.

At this point, the distal end 91 of the optical fiber bundle 9 points to the inner wall 161 of the blood vessel 16 so that the blood vessel inner wall 161 can be visualized through the light-transmitting and receiving fibers 10 and 11.

The inclination angle of the distal end 91 of the optical fiber bundle 9, that is, the visible direction may be varied by adjusting the distance of travel of the optical fiber bundle 9.

-18-

The bending section 32 of the first lumen 3 preferably has a length of 1 to 20 mm from the distal end of the tube body 2. The reason is that a bending section 32 of shorter than 1 mm cannot achieve a sufficient bend whereas it is difficult to form a bending section of longer than 20 mm.

The inclination angle β of the bending section 32 is preferably in the range of from 5 to 80°. The reason is that sometimes the blood vessel inner wall cannot be fully visualized at an angle β of less than 5 degrees whereas an angle β of more than 80 degrees would cause the optical fiber bundle to be bent at such a large angle that the optical fiber can be broken. The bending section 32 preferably has a length of about 2 to 5 mm and an inclination angle β of about 5 to 15° particularly when the catheter tube of the invention is applied as an endoscope for visualizing or medically treating a slender body cavity such as a blood vessel and ureter.

Although it is not critical how to form the bending section 32, the bending section is preferably manufactured by tip processing as described below because the bending section can be formed at the same time when the distal end of the tube is rounded. A core member (e.g., a metal core) having a previously bent distal portion is inserted into the first lumen 3 from the distal end of the tube body 2. The distal portion of the tube body is heated at a temperature of about 80 to 200°C, cooled down, and solidified, thereby forming the bending section 32 of the first lumen 3.

[Third Embodiment]

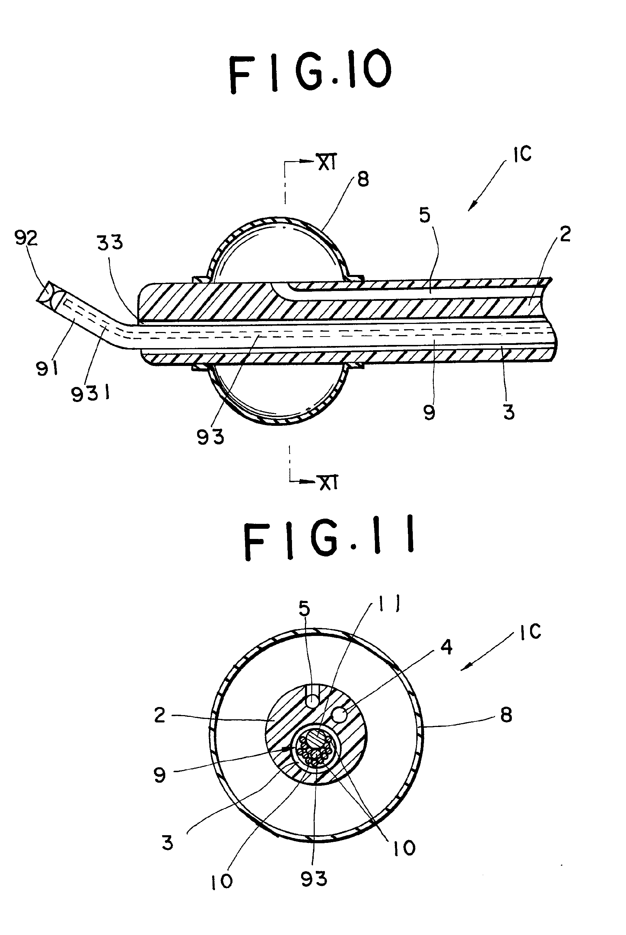

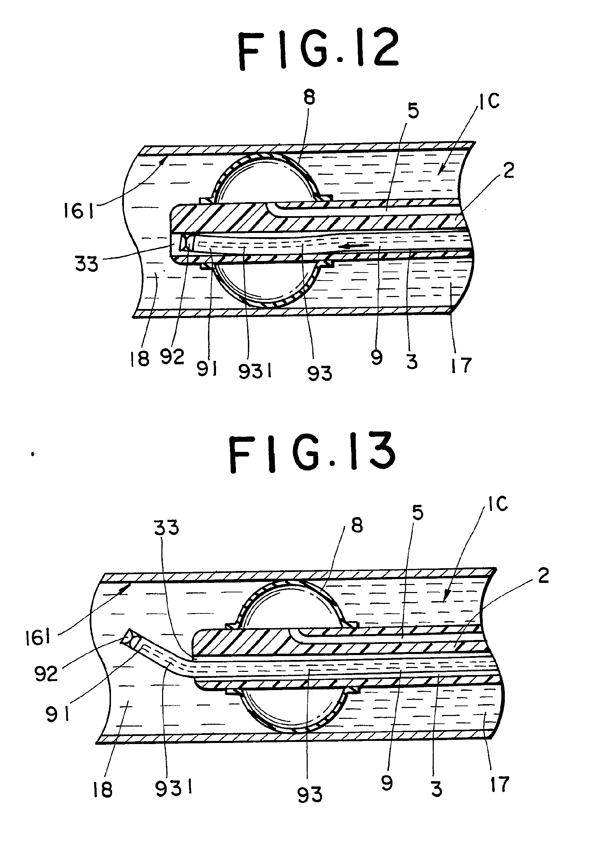

FIG. 10 is a fragmental axial cross-sectional view of a catheter tube according to a further embodiment of the present invention, and FIG. 11 is a cross-sectional view taken along lines XI-XI in FIG. 10.

FIGS. 12 and 13 are fragmental cross-sectional views showing the catheter tube 1C of FIG. 10 during its use.

In the description of the construction and operation of the catheter tube 1C shown in these figures, only the difference from the catheter tube 1A of the first embodiment is described because the remaining elements are the same.

The tube body 2 has a first lumen 3 defined therein. The first lumen 3 extends linearly to the distal end of the tube body 2 and opens at the distal end to define an opening 33.

An optical fiber bundle 9 is accommodated for movement in the first lumen 3. A core member 93 having a previously bent distal portion 931 is embedded in the optical fiber bundle 9. Then, when the distal portion 91 of the optical fiber bundle 9 is protruded from the opening 33 as shown in FIG. 10, the resiliency (or restoring force) of the distal portion 931 of the core member 93 causes the distal portion 91 of the optical fiber bundle 9 to bend at a predetermined angle.

The core member 93 may be selected from metal wires of stainless steel, carbon steel, hardened steel and phosphor bronze, and resin wires of polyurethane, polycarbonate, polysulfone, and polyamide. It may have a diameter of 0.1 to 1.0 mm, for example. The cross sectional shape of the core member 93 is circular in FIG. 11, but may assume an elliptical, rectangular, polygonal or any other shape.

The bendable distal portion 91 of the optical fiber bundle 9 preferably has a length of about 1 to 20 mm for the same reason as previously described.

The distal portion 91 of the optical fiber bundle 9 preferably has an average inclination angle of about 5 to

-20-

70° with respect to an axial direction of the tube body because breakage of optical fibers can be prevented.

The bendable portion 91 of the optical fiber bundle 9 preferably has a length of about 3 to 20 mm and an average inclination angle of about 5 to 45° particularly when the catheter tube of the invention is applied as an endoscope for visualizing or medically treating a slender body cavity such as a blood vessel and ureter.

The bending factors such as length and average inclination angle of the bendable distal portion 91 of the optical fiber bundle 9 may be properly determined through a proper choice of the material, shape and diameter of the core member 93 and the length and bent angle of the core distal portion 931 while taking into account the flexibility of the distal portion 91 of the optical fiber bundle 9.

The catheter tube 1C of such a construction operates as follows.

When the distal portion 91 of the optical fiber bundle 9 is retracted in the first lumen 3 as shown in FIG. 12, the inner wall of the first lumen 3 restrains bending of the distal portion 91 of the optical fiber bundle 9 although the previously bent distal portion 931 of the core member 93 forces bending. At this point, the inner wall of the first lumen 3 is subject to the reaction force so that the distal portion of the tube body 2 can be somewhat bent, but to a negligibly small extent. Such bending due to the reaction force can be avoided if the distal portion of the tube body 2 is formed of a hard material.

The optical fiber bundle 9 is then moved from this position toward the distal end of the tube body 9 as shown by an arrow by means of the moving manipulator 12 located at a proximal side of the tube body. When the distal portion 91 is protruded out of the opening 33, the restraint on the distal portion 91 is released so that the distal portion 91 may resume the bend.

At this point, the distal end 91 of the optical fiber bundle 9 points to the inner wall 161 of the blood vessel 16 so that the blood vessel inner wall 161 can be visualized through the light-transmitting and receiving fibers 10 and 11.

The inclination angle of the distal portion 91 of the optical fiber bundle 9, that is, the visible direction may be varied by adjusting the distance of travel of the optical fiber bundle 9.

When the lens 92 at the distal end of the optical fiber bundle 9 is located adjacent the opening 33 of the first lumen 3, the distal portion 91 assumes an inclination angle of approximately zero so that the lens 92 may point forward in the blood vessel 16.

The visible direction may be varied by rotating or twisting the optical fiber bundle 9 at its proximal end. This is effective in visualizing the blood vessel inner wall over its entire circumference. This leads·to an advantage that the blood vessel inner wall can be visualized over its entire circumference even when the blood flow is blocked by the balloon and the catheter tube is secured relative to the blood vessel, without a cumbersome need for contracting the balloon and rotating the catheter tube itself every time when it is desired to change the visible direction.

The catheter tube of the invention is not limited to the first, second and third embodiments described above insofar as it has bending means for allowing a distal portion of a visualizing or therapeutic instrument such as an optical fiber bundle to be bent without substantially bending a distal portion of the catheter tube.

Although the catheter tube having the balloon 8 and the third lumen for balloon inflation has been illustrated in the first, second and third embodiments, the invention is, of course, applicable to a catheter tube without a balloon and associated components.

Although visualization of the interior of the blood vessel through light-transmitting and receiving fibers has been described, the catheter tube 1 of the present invention is not limited to this application, but may be utilized in a wide variety of applications including administration of liquid medicament, irradiation of laser beams through optical fibers, tip guidance upon insertion to a destined site, and the like.

Examples of the present invention are given below by way of illustration.

Example 1

A catheter tube of the structure shown in FIGS. 1, 2, and 3 was manufactured. Various parameters of this catheter tube are described below.

<Tube body>

Material: polyvinyl chloride containing a radiopaque agent

Outer diameter: about 2.5 mm

Entire length: about 1.5 m

Lumen: 3 lumens

Clear liquid injecting lumen: 1

Fiber accommodating lumen: 1

Balloon inflating lumen: 1

<Optical fiber>

A single optical fiber bundle having an outer diameter of about 0.8 mm was prepared by integrally joining an image fiber (a bundle of about 2,000 quartz fibers of about 2-3 μm in diameter) and a light guide (a bundle of 25 quartz fibers of about 50 μm in diameter).

A convex lens was mounted on the end face of the image fiber such that the lens might receive the light emitted from the light guide and form the image of an object on the end face of the image fiber.

<Guide member>

A bar of polyvinyl chloride was beveled into a piece of an appropriate length, which was inserted into the fiber accommodating lumen from its distal end such that the beveled surface might form the ramp surface along which the tip of the optical fiber bundle might slide. The ramp surface had an angle α of 30°.

<Side port>

The outer peripheral wall of the tube body was formed with a side port having an open area of 5 mm$^2$ at a position corresponding to the ramp surface.

<Balloon>

Material: latex rubber

Thickness: about 150 μm

Shape: cylindrical

Effective length: 7 mm

Inflated diameter: 6 mm

<Catheter tube proximal side>

Fiber bundle manipulator: structure shown in FIG. 3

Female connector segment: entire length  40 mm

Male connector segment: entire length 40 mm

Maximum stroke: 20 mm

An eyepiece was mounted to the proximal end of the image fiber, enabling direct observation. The proximal end of the light guide was coupled to a light connector which was connected to a white light source.

The proximal end of the clear liquid injecting lumen was coupled to a cock having a Luer tapered socket, to which a syringe A was connected such that physiological saline could be supplied to the lumen.

The proximal end of the lumen leading to the balloon was coupled to a valve having a Luer tapered socket, to which a syringe B was connected such that inflating fluid (physiological saline) could be injected into the balloon.

The catheter tube of such construction was used to visualize a blood vessel.

First, the catheter tube was inserted into a blood vessel having an inner diameter of about 5 mm. The syringe B was manually operated, causing the balloon to inflate, thereby retaining the catheter tube relative to the blood vessel and shutting off a blood flow.

Then the syringe A was operated to inject 1.5 ml of physiological saline into the blood vessel forward of the balloon (on the distal side of the tube), thereby purging the blood therefrom. The volume of the blood vessel defined forward of the balloon was filled with the saline.

Next, the male connector segment of the manipulator was operated to move the optical fiber bundle 5 mm toward the distal end of the catheter tube whereby the distal end of the optical fiber bundle projected out of the side port.

The inner wall of the blood vessel could be observed by looking into the eyepiece or watching a video monitor screen connected in a well-known system, resulting in clear visualization without entry of blood into the zone under observation.

Example 2

A catheter tube of the structure shown in FIGS. 6 and 7 was manufactured. Various parameters of this catheter tube are described below.

<Tube body>

Material: polyvinyl chloride containing a radiopaque agent

Outer diameter: about 4.0 mm

Entire length: about 1.5 m

Lumen: 3 lumens

Clear liquid injecting lumen: 1

Fiber accommodating lumen: 1

Balloon inflating lumen: 1

The distal portion of the fiber accommodating lumen included a bending section extending 10 mm from the distal end and having an inclination angle β of 5°.

<Optical fiber>

The same as in Example 1.

<Balloon>

Material: latex rubber

Thickness: about 150 μm

Shape: cylindrical

Effective length: 10 mm

Inflated diameter: 10 mm

<Catheter tube proximal side>

The construction was the same as in Example 1 except that the balloon inflating fluid was changed to $CO_2$ gas.

The catheter tube of such construction was used to visualize a blood vessel.

First, the catheter tube was inserted into a blood vessel having an inner diameter of about 6 mm. The syringe B was manually operated, causing the balloon to inflate, thereby retaining the catheter tube relative to the blood vessel and shutting off a blood flow.

Then the syringe A was operated to inject 2.0 ml of physiological saline into the blood vessel forward of the balloon (on the distal side of the tube), thereby purging the blood therefrom. The volume of the blood vessel defined forward of the balloon was filled with the saline.

Next, the male connector segment of the fiber bundle moving manipulator was operated to move the optical fiber bundle 5 mm toward the distal end of the catheter tube whereby the distal end of the optical fiber bundle projected out of the opening of the fiber bundle accommodating lumen.

The inner wall of the blood vessel and the adjoining zone could be observed by looking into the eyepiece or watching a video monitor screen connected in a well-known system, resulting in clear visualization without entry of blood into the zone under observation.

It was confirmed on the monitor image that the visible field in the axial direction of the blood vessel could be varied by changing the distance of travel of the optical fiber bundle to adjust the inclination angle of the distal portion of the optical fiber bundle.

Example 3

A catheter tube of the structure shown in FIGS. 10 and 11 was manufactured. Various parameters of this catheter tube are described below.

<Tube body>

Material: polyvinyl chloride containing a radiopaque agent

Outer diameter: about 2.3 mm

Entire length: about 1.5 m

Lumen: 3 lumens

Clear liquid injecting lumen: 1

Fiber accommodating lumen: 1

Balloon inflating lumen: 1

<Optical fiber>

The same as in Example 1.

<Core member>

A stainless steel wire of 0.2 mm in diameter having a distal portion bent by a mechanical bending technique was assembled with the fibers to form an integral bundle.

The distal portion of the fiber bundle was bent at 30° with respect to the axis of the fiber bundle over a length of 20 mm from the distal end under normal conditions without any restraint.

<Balloon>

The same as in Example 1.

<Catheter tube proximal side>

The same as in Example 2.

The catheter tube of such construction was used to visualize a blood vessel.

First, the catheter tube was inserted into a blood vessel having an inner diameter of about 5 mm. The syringe B was manually operated, causing the balloon to inflate, thereby retaining the catheter tube relative to the blood vessel and shutting off a blood flow.

Then the syringe A was operated to inject 1.5 ml of physiological saline into the blood vessel forward of the balloon (on the distal side of the tube), thereby purging the blood therefrom. The volume of the blood vessel defined forward of the balloon was filled with the saline.

Next, the male connector segment of the fiber bundle moving manipulator was operated to move the optical fiber bundle 5 mm toward the distal end of the catheter tube whereby the distal end of the optical fiber bundle projected out of the distal opening of the fiber bundle accommodating lumen.

The inner wall of the blood vessel and the adjoining zone could be observed by looking into the eyepiece or watching a video monitor screen connected in a well-known system, resulting in clear visualization without entry of blood into the zone under observation.

It was confirmed on the monitor image that the visible field in the axial direction of the blood vessel could be varied by changing the distance of travel of the optical fiber bundle to adjust the inclination angle of the distal portion of the optical fiber bundle.

INDUSTRIAL APPLICABILITY

The invention provides a catheter tube wherein a distal portion of a linear visualizing or therapeutic instrument in the form of a bundle of optical fibers can be bent without substantially bending a distal portion of the catheter tube whereby the inner wall of a body cavity can be visualized or medically treated with a simple structure while eliminating any adverse influences of a complicated construction associated with provision of a tool, device or mechanism for bending the distal portion of the catheter tube and an increase in diameter of the catheter tube.

Since the catheter tube can be reduced in diameter for the simple structure and other reasons, it is advantageously applicable to body cavities with a smaller inner diameter, for example, blood vessels, ureter, and bile duct, especially as a blood vessel endoscope.

The catheter tube of the invention can accommodate for a change of the visible site or visible direction through a simple operation.

CLAIM:

1.    A catheter tube capable of accommodating therein a wire-like visualizing or therapeutic instrument, said catheter tube being provided with a bending mechanism for bending a distal portion of said visualizing or therapeutic instrument protruding from a distal portion of said catheter tube, without substantially bending the distal portion of said catheter tube.

2.    A catheter tube for use by inserting into a body cavity, comprising

    a tube body,

    a side port defined in the outer peripheral wall of said tube body adjacent a distal end thereof,

    a first lumen in flow communication with said side port,

    a visualizing or therapeutic instrument accommodated for movement in said first lumen,

    a guide member mounted in said first lumen of said tube body adjacent its distal end, said guide member having a ramp surface for guiding a distal portion of said visualizing or therapeutic instrument from within said first lumen to said side port when said visualizing or therapeutic instrument is moved toward the distal end of said tube body,

    a second lumen open at the distal end of said tube body, and

    a moving mechanism attached to a proximal portion of said tube body for axially moving said visualizing or therapeutic instrument through said tube body.

3.    The catheter tube of claim 2 wherein said ramp surface has an average inclination angle of 5 to 80° relative to the axial direction of said tube body.

4.   A catheter tube for use by inserting into a body cavity, comprising

a tube body,

a first lumen open at the distal end of said tube body, said first lumen at a distal portion thereof having a bending section which is inclined relative to the axial direction of said tube body,

a visualizing or therapeutic instrument accommodated for movement in said first lumen,

a second lumen open at the distal end of said tube body, and

a moving mechanism attached to a proximal portion of said tube body for axially moving said visualizing or therapeutic instrument through said tube body.

5.   The catheter tube of claim 4 wherein said bending section of said first lumen has a length of 1 to 20 mm from the distal end of said tube body.

6.   The catheter tube of claim 4 or 5 wherein said bending section of said first lumen has an average inclination angle of 5 to 80° relative to the axial direction of said tube body.

7.   A catheter tube for use by inserting into a body cavity, comprising

a tube body,

a first lumen open at the distal end of said tube body,

a visualizing or therapeutic instrument accommodated for movement in said first lumen, a distal portion of said visualizing or therapeutic instrument being bendable when the distal portion of said visualizing or therapeutic instrument is protruded from the open end of said first lumen,

a second lumen open at the distal end of said tube body, and

a moving mechanism attached to a proximal portion of said tube body for axially moving said visualizing or therapeutic instrument through said tube body.

8. The catheter tube of claim 7 wherein a core member having a previously bent distal portion is embedded in said visualizing or therapeutic instrument so that the distal portion of said visualizing or therapeutic instrument is bent.

9. The catheter tube of claim 7 or 8 wherein said visualizing or therapeutic instrument is bendable over a length of 1 to 20 mm from its distal end.

10. The catheter tube of any one of claims 2 to 9 wherein the inner surface of said first lumen has been subjected to lubricating treatment.

11. The catheter tube of any one of claims 1 to 10 wherein said visualizing or therapeutic instrument is a bundle of optical fibers.

12. The catheter tube of any one of claims 2 to 11 which further comprises at least one inflatable/contractible balloon attached around the outer peripheral wall of said tube body near its distal end, and a third lumen defined in said tube body in flow communication with said balloon.

13. The catheter tube of claim 12 wherein said balloon, when inflated, has a minimum diameter at least substantially equal to the maximum inner diameter of the body cavity into which the catheter tube is inserted.

# FIG. 1

# FIG. 2

# F I G. 3

# FIG.4

# FIG.5

# FIG.6

# FIG.7

# FIG.8

# FIG.9

# FIG. 10

# FIG. 11

# FIG.12

# FIG.13

# INTERNATIONAL SEARCH REPORT

International Application No  PCT/JP88/00860

## I. CLASSIFICATION OF SUBJECT MATTER (if several classification symbols apply, indicate all) [6]

· According to International Patent Classification (IPC) or to both National Classification and IPC

Int.Cl$^4$    A61B1/00

## II. FIELDS SEARCHED

### Minimum Documentation Searched [7]

| Classification System | Classification Symbols |
|---|---|
| IPC | A61B1/00, A61M25/00 |

### Documentation Searched other than Minimum Documentation to the Extent that such Documents are Included in the Fields Searched [8]

| | |
|---|---|
| Jitsuyo Shinan Koho | 1968 – 1988 |
| Kokai Jitsuyo Shinan Koko | 1971 – 1988 |

## III. DOCUMENTS CONSIDERED TO BE RELEVANT [9]

| Category* | Citation of Document, [11] with indication, where appropriate, of the relevant passages [12] | Relevant to Claim No. [13] |
|---|---|---|
| X | JP, B2, 57-810 (Fuji Photo Optical Co., Ltd.) 8 January 1982 (08. 01. 82) Column 2, lines 11 to 21, Fig. 1 (Family: none) | 1 |
| Y | DE, C2, 2848484 (Olympus Optical Co., Ltd.) 10 May 1979 (10. 05. 79) Column 4, lines 23 to 43 (Family: none) | 1-3 |
| Y | JP, A, 54-136780 (Olympus Optical Co., Ltd.) 24 October 1979 (24. 10. 79) Figs. 8 to 11 (Family: none) | 1-7 |
| Y | JP, U, 57-130605 (Olympus Optical Co., Ltd.) 14 August 1982 (14. 08. 82) Figs. 2, 6 (Family: none) | 1-7 |
| Y | JP, U, 58-171103 (Medosu Kenkyusho Kabushiki Kaisha) 15 November 1983 (15. 11. 83) Figs. 5, 6 (Family: none) | 7 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| November 10, 1988 (10. 11. 88) | November 21, 1988 (21. 11. 88) |

| International Searching Authority | Signature of Authorized Officer |
|---|---|
| Japanese Patent Office | |